# EUROPEAN PATENT SPECIFICATION

(11) **EP 3 292 236 B1**
(45) Date of publication and mention of the grant of the patent: **24.04.2019**
(21) Application number: 16725047.1
(22) Date of filing: 04.05.2016
(51) Int. Cl.: D04H 1/4391, A61F 13/51, D04H 1/544, D04H 3/018, D04H 3/14

(54) **NON-WOVEN**
VLIESSTOFF
ÉTOFFE NON TISSÉE

(30) Priority: 06.05.2015 EP 15166616
(43) Date of publication of application: 14.03.2018
(73) Proprietor: Fitesa Germany GmbH, 31224 Peine (DE)
(72) Inventor: SIEBNER, Harald, 38122 Braunschweig (DE); HARTL, Helmut, 38124 Braunschweig (DE); NOVARINO, Elena, 31224 Peine (DE)
(74) Representative: van Wijk, Alexander Pieter
(86) International application number: PCT/EP2016/060013
(87) International publication number: WO 2016/177787

(56) References cited:
- EP-A1- 1 722 020
- EP-A1- 2 821 043
- WO-A1-2012/125701
- WO-A1-2012/130414
- CN-U- 201 785 556
- DE-A1- 3 634 146
- US-A- 3 508 390
- US-A- 5 208 106
- US-A1- 2004 092 192
- US-A1- 2008 032 579

## Description

### FIELD OF THE INVENTION

The present invention relates to a nonwoven fabric, an absorbent article comprising the non-woven fabric, the use of the nonwoven fabric in a closure system in an absorbent article or in a reinforcement layer of an absorbent article, and an absorbent article comprising the nonwoven fabric.

### BACKGROUND OF THE INVENTION

Nonwoven fabrics are widely applied in disposable absorbent articles for personal care or hygiene, such as diapers, feminine care articles, incontinence articles and children's training pants. A particular category amongst such absorbent articles are adult incontinence articles which comprise an absorbent pad, a frontal ear and a waist belt that needs to be attached around the waist of the adult wearer. The materials of which waist belts and frontal ear components in adult incontinence articles are made need to meet a number of criteria to ensure that wearing comfort is established for the adult wearer. One critical factor is that these materials need to have sufficient stiffness to avoid excessive wrinkling and twisting of the absorbent article. Another critical factor is that the materials display sufficient softness to avoid skin problems associated with cutting and abrasion. Moreover, the appearance of softness is of great importance in such articles since it reassures the wearer that the article will be experienced as comfortable.

In EP 2821043 A1, nonwoven fabrics are described which are formed from fibers that preferably have a round cross-section. Such nonwoven fabrics have the drawback, however, that their stiffness leaves considerable room for improvement, particularly at lower basis weights..

Object of the present invention is to provide a nonwoven fabric from which components for absorbent articles, in particular adult incontinence articles, can be made which display an improved stiffness and at the same time establish improved wearer comfort.

### SUMMARY OF THE INVENTION

It has now been found that this can be established when use is made of nonwoven fabrics comprising a nonwoven web which is formed from a particular type of fibers.

Accordingly, the present invention relates to nonwoven fabric as defined in claim 1.

An advantage of the present invention resides in the fact that the present nonwoven fabric enables the manufacture of adult incontinence articles which display enough strength to fix the article properly around the waist of the wearer, whereas at the same time the wearer will experience improved wear comfort. Moreover, the present nonwoven fabrics make it possible to produce articles in which the waist belt and the outer side of the article are made of the same fiber materials, contributing to the experience of wearer comfort. Another major advantage of the present nonwoven fabrics is that they provide higher stiffness at lower basis weights. Further, due to the use of the particular fibers no further measures are needed to stiffen the nonwoven fabrics. This means for instance that the nonwoven fabrics do not need a separate meltblown layer or the addition of polyesters to provide sufficient support. Hence, the nonwoven fabrics in accordance with the present invention bring about considerable cost reductions when compared with the use of conventional nonwoven fabrics in absorbent articles, in particular adult incontinence articles. Moreover, the recycling of these articles is very much improved since no separate meltblown layers or polyesters are needed to provide sufficient stiffness to the nonwoven fabrics.

A major advantage of the present invention resides in the fact that the present nonwoven fabric displays an improved stiffness when compared with nonwoven fabrics that are formed from round fibers which are conventionally used.

### DETAILED DESCRIPTION OF THE INVENTION

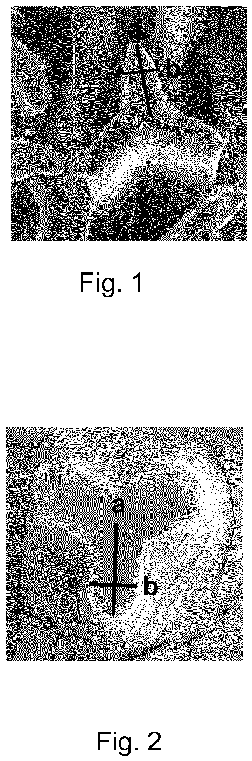

In accordance with the present invention the nonwoven fabric comprises a nonwoven web which is formed from a plurality of trilobal fibers, wherein the lobes of the trilobal fibers each have a ratio of length to width (L/W) in the range of from 2.2-4, and preferably 2.5-4. In the context of the present invention the length of a lobe means the distance between the end point of a lobe and the center of the core of the trilobal fiber (see also lines (a) in Figures 1 and 2). The width is defined as the average distance between the two opposite (outer) sides of an individual lobe (see also lines (b) in Figures 1 and 2).

Suitably, in accordance with the present invention the trilobal fibers are made from thermoplastic polymers. The nonwoven webs particularly suitable are made from fibers of thermoplastic polymers such as polyolefins, polyesters, ethylene copolymers, propylene copolymers, butene copolymers, and combinations thereof, but may also comprise natural fibers such as wood, cotton, or rayon in combination with thermoplastic fibers. The nonwoven web may also be a composite made up of a mixture of two or more different fibers or a mixture of fibers and particles.

The fibers are suitably joined by bonding to form a coherent web structure. Suitable bonding techniques include, but are not limited to, chemical bonding and thermal bonding, for example thermal calendering or bonding by a hot gas stream.

A wide range of suitable polyolefins can be used in the present invention. Suitable examples include polyethylene, polypropylene, copolymers of ethylene and butene, hexene, or octene, copolymers of polypropylene and ethylene. The polyolefins may comprise a homopolymer or a copolymer such as propylene-α-olefins copolymers. In particularly, the latter copolymers can attractively be used in the present invention. Preferred are polyolefin materials that comprise a propylene-α-olefin copolymer and a propylene homopolymer.

The melt flow rate (MFR) of the polyolefin material is suitably less than 90 dg/min. The MFR is determined using ASTM test method D1238, 2.16 kg. Preferably, the MFR of the polyolefin material is in the range of from 15-50 dg/min, more preferably in the range of from 15-35 dg/min.

Preferably, the fibers are formed from polyethylene or polypropylene homopolymers, co-polymers thereof, blends of polyethylene and polypropylene, a polyester, co-polymers of polyesters and/or blends of polyesters.

Suitably use is made of a propylene-based or ethylene-based homopolymer or a copolymer. In the case of propylene-based polymers the polymers may comprise comonomer-derived units selected from ethylene and C4-C10 α-olefins. In the case of ethylene-based polymers the polymers may comprise comonomer-derived units selected from C3-C10 α-olefins. Suitable examples of polyolefin materials include propylene homopolymers, ethylene homopolymers, propylene copolymers and ethylene copolymers such as linear low density polyethylene (LLDPE), high density polyethylene (HDPE), and low density polyethylene (LDPE).

Suitable polyesters can be aliphatic polyesters such as, e.g. polylactic acid, or aromatic polyesters such as polyethylene terephthalate (PET) and poly(trimethylene terephthalate (PTT).

Besides additives already contained in the employed polymers, addition of further additives is possible to provide additional properties to the fibers. Suitable further additives include thermal stabilizers, light stabilizers, slip additives, waxes, and additives to make the fabrics either hydrophilic or hydrophobic. The addition of filler materials can sometimes also be of advantage. Suitable filler materials include organic and inorganic filler materials. Suitable examples of inorganic filler materials include minerals such as calcium carbonate, metals such as aluminium and stainless steel. Suitable examples of organic filler materials include sugar-based polymers. The trilobal fibers to be used in accordance with the present invention are bicomponent fibers. The lobes are made of one type of polymer and the core to which the lobes are attached will be made of another type of polymer. Most preferred are core-sheath bicomponent fibers comprising polyethylene and polypropylene, but any other combination of other suitable polymers is possible as well, for example combinations of polyesters with polyolefins. The bicomponent fibers may contain different types of polypropylene. More preferably, the bicomponent fiber has a core of a polypropylene which has a higher melting point and lobes of a polypropylene which has a lower melting point. In another embodiment, the bicomponent fiber comprises two polypropylenes that differ in melt temperature or melt flow.

The fibers can be made according to spinning technologies known in the art. Most conveniently employed are spunbond processes, from which the nonwoven fabrics can directly be formed.

Spunbond fibers are generally produced by extruding a molten polymer through a large spinneret having several thousand holes per linear meter or from banks of smaller spinnerets, for example, containing as few as 40 holes. After exiting the spinneret, the molten fibers are quenched by a cross-flow air quench system, then pulled away from the spinneret and attenuated by high speed air. Lay-down of the filaments to create a nonwoven layer occurs on a permeable transport belt. Spunbond fibers are generally continuous and range in fiber diameter between ca. 10-100 µm.

The non-woven fabric in accordance with the invention can additionally be treated to add specific properties. Most common are topical treatments to make the fabric either hydrophilic or to make it hydrophobic. Most common is the treatment of the fabric with either hydrophilic surfactants or with a fluorocarbon or a silicon material. In the context of the present invention a surface of a non-woven fabric or non-woven web is "hydrophilic" when the contact angle of water disposed on that surface is less than about 90 and a surface is "hydrophobic" when the contact angle of water disposed on that surface is greater than or equal to 90.

Preferably, the nonwoven fabrics in accordance with the invention are hydrophobic non-woven fabrics.

The nonwoven fabric according to the invention can consist of only one type of fibers or fiber layers, e.g. a spunbond layer, but it suitably can comprise additional fiber layers which may be different. Suitable multi-layer fabrics, for example, may include one or more spunbond layers (S) and one or more meltblown layers (M), such as SMS, SMMS, SSMMS, etc. adhered to form a nonwoven fabric according to the present invention. Usually, these multilayer fabrics are made in one step on a single line with multiple beams, which generally encompass a combination of spunbond and meltblown beams. In some cases it might be advantageous or technically necessary to make a multiple layer according to the invention in two or more separate steps. A major advantage of the present invention is however that the nonwoven fabrics of the present invention do not require additional fiber layers such as meltblown layers to increase their stiffness.

Combination of spunbond layers with natural fibers is possible as well. Preferably, the additional nonwoven webs to be used in accordance with the present invention are made of meltblown fibers.

Suitably, the nonwoven web according to the present invention has a tensile strength according WSP 110.4 in MD (Machine Direction) in the range of from 1-4 N per gram basis weight, preferably in the range of from 1.2-3.5 N per gram basis weight, and more preferably in the range of from 1.3-3.0 N per gram basis weight. Non-woven webs with such tensile strengths provide non-woven articles with a high tensile strength.

The skilled person will understand that the major part of the nonwoven fabric will consist of the fibers. Suitably, at least 90 wt% of the nonwoven fabric is made of fibers, preferably at least 95 wt% of the nonwoven fabric is made of fibers, and even more preferably at least 98 wt% of the nonwoven fabric is made of fibers.

Suitably, the nonwoven web has a basis weight in the range of 10-20 gsm (g/m²).

This WSP test method is an internationally acknowledged test method in the non-woven's industry, as the skilled person will understand.

The present nonwoven fabrics are suitably made from fibers that have a weight in the range of from 1-6 dtex, preferably in the range of from 1.5-5 dtex, and more preferably in the range of from 1.8-4 dtex.

Suitably, the nonwoven webs to be used in accordance with the present invention comprise at most 25 wt% of meltblown fibers, based on total weight of the non-woven web. Preferably, the non-woven webs comprises at most 20 wt% of meltblown fibers, based on the total weight of the non-woven web.

Suitably, the present nonwoven webs contain spunbond fibers only, not mixtures of spunbond fibers and another type of fibers.

The nonwoven fabrics in accordance with the present invention comprise a nonwoven web which has a side which is provided with a pattern of bonded areas which defines one or more non-bonded areas, and wherein the surface of the bonded areas is in the range of 10-32 % of the total surface of the side and the surface of the non-bonded area(s) is in the range of from 68-90 % of the total surface of the side.

Therefore, the present invention also relates to a nonwoven fabric comprising a nonwoven web which is formed from a plurality of trilobal fibers, wherein the lobes of the trilobal fibers each have a ratio of length to width in the range of from 2.2-4, wherein the nonwoven web which has a side which is provided with a pattern of bonded areas which defines one or more non-bonded areas, and wherein the surface of the bonded areas is in the range of 10-32 % of the total surface of the side and the surface of the non-bonded area(s) is in the range of from 68-90 % of the total surface of the side. The nonwoven web has a side which is provided with a first pattern of individualized bonded areas and a second pattern of non-bonded areas, and wherein the surface of the bonded areas is in the range of 10-32 % of the total surface of the side and the surface of the non-bonded areas is in the range of from 68-90 % of the total surface of the side.

The present invention therefore also provides a nonwoven fabric comprising a nonwoven web which is formed from a plurality of trilobal fibers, wherein the lobes of the trilobal fibers each have a ratio of length to width in the range of from 2.2-4, wherein the nonwoven web which has a side which is provided with a first pattern of individualized bonded areas which define a second pattern of non-bonded areas, and wherein the surface of the bonded areas is in the range of 10-32 % of the total surface of the side and the surface of the non-bonded areas is in the range of from 68-90 % of the total surface of the side.

The trilobal fibers to be used according to the present invention allow a more open bond pattern (lower bond area) when compared with the denser bond patterns that are obtained with for instance round fibers, whilst achieving the same degree of required stiffness. In addition, the higher stiffness of the present fibers does advantageously not directly translate into a lower softness of the fabric. To the contrary, the more open bond pattern that is established with the present invention, allows for higher bulkiness and fluffiness, which results in an increased perceived softness.

The high surface of the non-bonded area(s) to be used according to the present invention provides an attractively high softness. Moreover, the large unbonded area(s) allow for the fiber to bulk up and increase the bulkiness of the fabric. This is perceived as an even higher softness from both visual and the tactile perspective. Preferably, the surface of the non-bonded area(s) is at least 68% and less than 90% of the total surface area of the side. Preferably, the surface of the non-bonded area(s) is in the range of from 75-85% of the total surface area of the side.

The surface of the bonded areas is preferably more than 10% and at most 32%, more preferably in the range of from 15-25 % of the total surface area of the side.

In a very preferred embodiment, a first pattern of individualized bonded areas defines a second pattern of non-bonded areas which non-bonded areas have a hexagonal type of shape.

Preferably, the side of the non-woven fabric is only provided with the first pattern and the second patterns, meaning that no further pattern of bonded or non-bonded areas is provided on the side of the non-woven fabric.

In nonwoven fabrics not according to the invention, the individualized bonded areas can be uniformly arranged over the total surface of the side of the nonwoven web. Such patterns are used for bonding since many years and are known to the person skilled in the art.

The individualized bonded areas suitably have a symmetrical shape. According to the invention the individualized bonded areas have a rod shape in the cross direction (CD) of the nonwoven web.

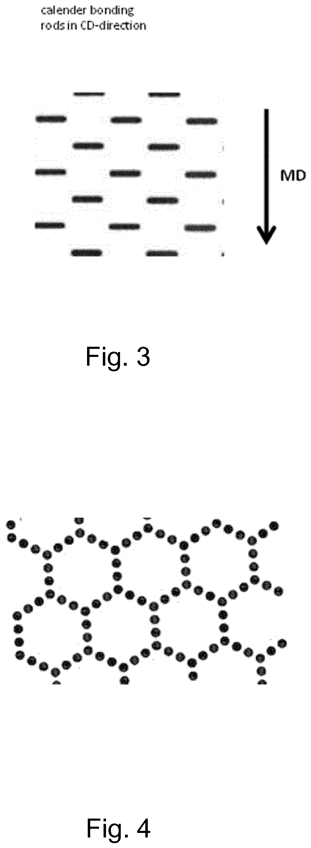

In nonwoven fabrics not according to the present invention, the pattern of bonded areas defines one large non-bonded area. This is for instance the case when the bonded areas have all the same shape and size and are all evenly distributed over the side of the nonwoven web. For instance, the bonded areas may have rod shapes in the cross direction (see Figure 3). According to the present invention, the pattern of bonded areas defines a pattern of a number of non-bonded areas, whereby the non-bonded areas may have various shapes. Suitable shapes of the non-bonded areas include squares, circles, and hexagons. Suitably, the non-bonded areas have the shape of a regular or an irregular hexagon in which one or more sides have a different length. Preferably, the non-bonded areas have a regular hexagonal shape (see Figure 4)..

Suitably, the non-bonded areas have a surface in the range of from 20-50 mm², preferably in the range of from 22-45 mm², and more preferably in the range of from 23-40 mm².

The bonded areas suitably have a maximum width in the range of from 0.7-1.5 mm, preferably in the range of from 0.75-1.25 mm, and more preferably in the range of from 0.8-1.2 mm.

Suitably, the bonded areas have a surface in the range of from 0.38-1.77 mm², preferably in the range of from 0.44-1.22 mm², and more preferably in the range of from 0.50-1.13 mm². The discrete non-bonded area(s) suitably have a depth in the range of from 0.4-1.5 mm, preferably in the range of from 0.4-0.9 mm, more preferably in the range of from 0.4-0.8 mm, and most preferably in the range of from 0.5-0.7 mm.

Suitably, an even number of bonded areas defines an individual non-bonded area. Preferably, the individual non-bonded areas are defined by 6, 12, 18 or 24 individualized bonded areas, more preferably 12, 18 or 24 individualized bonded areas, and most preferably by 12 individualized bonded areas.

The present invention also relates to an absorbent article comprising a nonwoven fabric according to the present invention. Suitably, the absorbent article according to the present invention is a disposable hygiene absorbent article selected from the group consisting of incontinence articles, diapers, wipes and fem-care articles. Suitable disposable hygiene absorbent articles according to the present invention include those selected from the group consisting of baby diapers, pull-ups, training pants, hygiene closure systems, adult incontinence briefs and diapers. Preferably, the absorbent article in accordance with the present invention is an adult incontinence article.

The nonwoven fabric according to the present invention can suitably be part of a top sheet, back sheet, landing zone and/or a waist belt, wing or a frontal ear. Preferably, the present nonwoven fabric is part of a closure system in adult incontinence article, preferably a waist belt, wing or frontal ear.

The present invention also relates to the use of the nonwoven fabric according to the present invention in a closure system in an absorbent article. Preferably, the closure system comprises a waist belt, frontal ear or wing.

The present invention further relates to the use of the nonwoven fabric according to the present invention in a reinforcement layer of an absorbent article.

In Figures 1 and 2, pictures are shown of trilobal fibers. In Figure 1, a trilobal fiber according to the invention is shown with a ratio of length to width of 1:3, whereas in Figure 2 a trilobal fiber is shown with a ratio of length to width of 1:2 (not according to the invention).

### Examples

In these Examples a comparison is made between nonwoven fabrics made from round fibers and nonwoven fabrics made from trilobal fibers. The stiffness of the two respective nonwoven fabrics is compared, whereby the stiffness is expressed in terms of bending length. The bending length is measured according to WSP 90.5.

### Example 1

In this Example, the surface of the bonded areas for both the nonwoven fabrics made from round fibers and trilobal fibers was 10.9 % and the surface of the non-bonded areas was 89.1 %, based on the total surface of one of the sides of the nonwoven fabrics. Further, in both cases the fiber titer was 2.2 dtex, and both nonwoven fabrics had a basis weight of 13 gsm. In Table 1, the stiffness in terms of bending length and the thickness of the fibers is indicated. The thickness of the round fibers is defined as the diameter of the fiber, whereas the thickness of the trilobal fibers is defined as the distance between the end points of two respective lobes.

**Table 1**

| **13 gsm** | Round fiber fabric | Trilobal fiber fabric |
|---|---|---|
| Bending length | 17 mm | 25 mm |
| Thickness nonwoven fabric (bulk) | 300 µm | 597 µm |
| Fiber thickness | 16 µm | 26 µm |

### Example 2

In this Example, the surface of the bonded areas for both the nonwoven fabrics made from round fibers and trilobal fibers had a bonded area of 18.1 % and the surface of the non-bonded area was 81.9 %, based on the total surface of one of the sides of the nonwoven fabrics. Further, in both cases the fiber titer was 2.2 dtex, and both nonwoven fabrics had a basis weight of 17 gsm. In Table 2, the stiffness in terms of bending length and the thickness of the fibers is indicated. The thickness of the round fibers is defined as the diameter of the fiber, whereas the thickness of the trilobal fibers is defined as twice the distance between the end point of a single lobe and the center of the core of the fiber.

**Table 2**

| **17 gsm** | Round fiber fabric | Trilobal fiber fabric |
|---|---|---|
| Bending length | 25 mm | 33 mm |
| Thickness nonwoven fabric (bulk) | 317 µm | 397 µm |
| Fiber thickness | 16 µm | 26 µm |

From Tables 1 and 2 it will be clear that the, nonwoven fabrics made from round fibers need a higher basis weight as well as higher bond area to establish a bending length of 25mm when compared with nonwoven made from triblobal fibers. Actualy, to achieve a bending length of 25 mm, a round fiber fabric needs 17 gsm and a bonded area of 18.1% while a trilobal fabric achieves same bending length with already 13 gsm and a bonding area of 10.9%. Moreover, the nonwoven fabric made from trilobal fibers had a bulkiness in terms of fabric thickness which was approximately twice as high when compared with the bulkiness of the nonwoven fabric made from the round fibers. In light of these findings it will be clear that the nonwoven fabrics according to the present invention constitute a major improvement over conventional nonwoven fabrics which are made from round fibers.

## Claims

1. A nonwoven fabric comprising a nonwoven web which is formed from a plurality of trilobal fibers, wherein the lobes of the trilobal fibers each have a ratio of length to width in the range of from 2.2-4, the nonwoven web has a basis weight in the range of from 10-20 gsm, wherein the trilobal fibers are bicomponent fibers of which the lobes are made of one type of polymer and the core to which the lobes are attached is made of another type of polymer, wherein the nonwoven web has a side which is provided with a first pattern of individualized bonded areas which defines a second pattern of non-bonded areas, wherein the individualized bonded areas have a rod shape in the cross direction (CD) of the nonwoven web, and wherein the surface of the bonded areas is in the range of 10-32 % of the total surface of the side and the surface of the non-bonded area(s) is in the range of from 68-90 % of the total surface of the side.

2. The nonwoven fabric according to claim 1, wherein the lobes of the trilobal fibers each have a ratio of length to width in the range of from 2.5-4.

3. The nonwoven according to claim 1 or 2, wherein the trilobal fibers are made from thermoplastic polymers.

4. The nonwoven according to claim 3, wherein the thermoplastic polymers comprise polypropylene and/or polyethylene.

5. The nonwoven fabric according to any one of claims 1-4, wherein the trilobal fibers have a weight in the range of from 1.5-5 dtex.

6. Use of the nonwoven fabric according to any one of claims 1-5 in a closure system in an absorbent article.

7. Use according to claim 6, wherein closure system comprises a waist belt, frontal ear or wing.

8. Use of the nonwoven fabric according to any one of claims 1-5 in a reinforcement layer of an absorbent article.

9. An absorbent article comprising a nonwoven fabric according to any one of claims 1-5.

10. An absorbent article according to claim 9, wherein the article is an adult incontinence article.

## Patentansprüche

1. Vliesstoff, das ein Vliesgewebe umfasst, das aus einer Mehrzahl von trilobalen Fasern gebildet ist, wobei die Lappen der trilobalen Fasern jeweils ein Verhältnis von Länge zu Breite im Bereich von 2,2 bis 4 aufweisen, das Vliesgewebe ein Grundgewicht im Bereich von 10 bis 20 g/m² aufweist, wobei die trilobalen Fasern Zweikomponentenfasern sind, deren Lappen aus einem Polymertyp hergestellt sind, und wobei der Kern, an dem die Lappen angebracht sind, aus einem anderen Polymertyp hergestellt ist, wobei das Vliesgewebe eine Seite aufweist, die mit einem ersten Muster aus einzelnen gebundenen Flächen versehen ist, das ein zweites Muster aus ungebundenen Flächen definiert, wobei die einzelnen gebundenen Flächen eine Stabform in der Querrichtung (CD) des Vliesgewebes aufweisen, und wobei die Oberfläche der gebundenen Flächen im Bereich von 10 bis 32 % der Gesamtfläche der Seite liegt und die Oberfläche des einen oder der mehreren ungebundenen Flächen im Bereich von 68 bis 90 % der Gesamtoberfläche der Seite liegt.

2. Vliesstoff nach Anspruch 1, wobei die Lappen der trilobalen Fasern jeweils ein Verhältnis von Länge zu Breite im Bereich von 2,5 bis 4 aufweisen.

3. Vliesstoff nach Anspruch 1 oder 2, wobei die trilobalen Fasern aus thermoplastischen Polymeren hergestellt sind.

4. Vliesstoff nach Anspruch 3, wobei die thermoplastischen Polymere Polypropylen und/oder Polyethylen umfassen.

5. Vliesstoff nach einem der Ansprüche 1 bis 4, wobei die trilobalen Fasern ein Gewicht im Bereich von 1,5 bis 5 dtex aufweisen.

6. Verwendung des Vliesstoffs nach einem der Ansprüche 1 bis 5 bei einem Verschlusssystem bei einem absorbierenden Gegenstand.

7. Verwendung nach Anspruch 6, wobei das Verschlusssystem einen Leibgurt, ein vorderes Ohr oder einen Flügel umfasst.

8. Verwendung des Vliesstoffs nach einem der Ansprüche 1 bis 5 in einer Verstärkungsschicht eines absorbierenden Gegenstands.

9. Absorbierender Gegenstand, der einen Vliesstoff nach einem der Ansprüche 1 bis 5 umfasst.

10. Absorbierender Gegenstand nach Anspruch 9, wobei der Gegenstand ein Inkontinenzgegenstand für Erwachsene ist.

## Revendications

1. Étoffe non tissée comprenant un voile non tissé qui est formé à partir d'une pluralité de fibres trilobées, les lobes des fibres trilobées ayant chacun un rapport longueur/largeur dans la plage allant de 2,2 à 4, le voile non tissé ayant un poids de base dans la plage allant de 10 à 20 g/m², les fibres trilobées étant des fibres à deux composants dont les lobes sont faits d'un premier type de polymère et l'âme, à laquelle les lobes sont attachés, est faite d'un autre type de polymère, le voile non tissé ayant un côté qui comporte un premier motif de zones liées individualisées qui définit un second motif de zones non liées, les zones liées individualisées ayant une forme de tige dans le sens travers (CD) du voile non tissé, et la surface des zones liées étant dans la plage allant de 10 à 32 % de la surface totale du côté et la surface de la ou des zones non liées étant dans la plage allant de 68 à 90 % de la surface totale du côté.

2. Étoffe non tissée selon la revendication 1, dans laquelle les lobes des fibres trilobées ont chacun un rapport longueur/largeur dans la plage allant de 2,5 à 4.

3. Étoffe non tissée selon la revendication 1 ou 2, dans laquelle les fibres trilobées sont faites à partir de polymères thermoplastiques.

4. Étoffe non tissée selon la revendication 3, dans laquelle les polymères thermoplastiques comprennent du polypropylène et/ou du polyéthylène.

5. Étoffe non tissée selon l'une quelconque des revendications 1 à 4, dans laquelle les fibres trilobées ont un poids dans la plage allant de 1,5 à 5 dtex.

6. Utilisation de l'étoffe non tissée selon l'une quelconque des revendications 1 à 5 dans un système de fermeture dans un article absorbant.

7. Utilisation selon la revendication 6, dans laquelle le système de fermeture comprend une ceinture ventrale, une oreille avant ou une ailette.

8. Utilisation de l'étoffe non tissée selon l'une quelconque des revendications 1 à 5 dans une couche de renfort d'un article absorbant.

9. Article absorbant comprenant une étoffe non tissée selon l'une quelconque des revendications 1 à 5.

10. Article absorbant selon la revendication 9, l'article étant un article d'incontinence pour adultes.
